# EUROPEAN PATENT SPECIFICATION

(11) **EP 2 726 036 B1**
(45) Date of publication and mention of the grant of the patent: **10.06.2015**
(21) Application number: 12733204.7
(22) Date of filing: 29.06.2012
(51) Int. Cl.: A61F 13/08, A61F 13/00, A61F 13/02, A61F 13/15, D04H 1/46, D04H 13/00, D04H 18/02, D04H 1/593, D04H 3/105, B32B 7/08

(54) **COMPRESSION BANDAGES AND METHOD OF MAKING THE SAME**
KOMPRESSIONSBANDAGEN UND VERFAHREN ZU IHRER HERSTELLUNG
BANDAGES COMPRESSIFS ET LEUR PROCÉDÉ DE FABRICATION

(30) Priority: 29.06.2011 GB 201111059
(43) Date of publication of application: 07.05.2014
(73) Proprietor: Lantor (UK) Limited, Bolton Lancashire BL3 3PP (GB); Karl Otto Braun GmbH & Co. KG., 67752 Wolfstein (DE)
(72) Inventor: RICHARDSON, David William, Bolton BL3 3PP (GB); STEPHENSON, Christian, Wilmslow Cheshire SK9 2EP (GB); WARDE, David Michael, Manchester M9 7BN (GB); MEISTER, Marita, 67657 Kaiserslautern (DE); KLÖPPELS, Michael Peter Wilhelm, 52064 Aachen (DE)
(74) Representative: DREISS Patentanwälte PartG mbB
(86) International application number: PCT/GB2012/051528
(87) International publication number: WO 2013/001312

(56) References cited:
- US-A- 3 482 570
- US-A- 4 446 189
- US-A1- 2009 221 946

## Description

The present invention relates to two layer compression bandages comprising a padding layer and an elastic compression layer. The invention further relates to the use of said bandages in the management and/or treatment of wounds or other lesions, for example venous leg ulceration. The invention also relates to processes and machinery for making such compression bandage systems.

Compression bandages are used to facilitate healing. For example, compression therapy is used to provide a graduated external compression to the lower limb, due to its ability to minimise or reverse the skin and vascular changes caused by venous leg disease.

A number of compression bandages have been described. For example, four-layer bandaging, a high-compression bandaging system that incorporates elastic layers to achieve a sustained level of compression over time, and is widely accepted as an effective treatment for venous leg ulcers. Typically, the four-layer compression bandages consist of:
i) Padding bandage - this layer is in direct contact with skin, is soft and comfortable, highly absorbent, and protects areas at risk of high pressure, such as bony prominences, on the foot and ankle.
ii) Crepe bandage - this layer simply provides extra absorbency and smoothes down the padding layer
iii) Elastic extensible bandage - this is a highly extensible bandage that when applied to the wound in a pre-determined manner provides graduated compression to the lower limb.
iv) Elastic cohesive bandage - this layer provides additional compression, maintains the bandage in position, and provides a protective layer.

However, four-layer compression therapy has disadvantages, such as the number of layers required make the therapy bulky, which can reduce patient mobility, be restrictive (clothing & footwear), and be aesthetically displeasing. A compression therapy system that uses fewer layers would mitigate these disadvantages. Furthermore a system that used fewer layers is likely to require fewer raw materials, and be easier (less layers means less chance of applying a layer incorrectly) and less time consuming to apply.

Another known bandage is the KTwo compression system made by Urgo Medical. This comprises a first layer (referred to a K Tech) and a second layer (referred to as K Press). The K Tech is a composite layer formed by wadding and a moderately elastic compression fabric, In the K Tech product the two layers can be separated easily by hand which is not ideal for a medical bandage. Therefore, there is a need for improved bandage materials. Bandages of the invention described below do not have this problem, the layers cannot be separated without destroying the material.

US 2009/0221946 discloses a multilayer wound dressing which comprises two absorbent needle-punched fibre batts which may be further bonded together via needling to a third absorbent layer of cotton gauze.

The present invention relates to a two layer compression bandage comprising a padding layer and an elastic layer in which the padding layer is combined with an elastic layer in such a way that the padding layer is fixed to the elastic bandage without undue reduction of the elastic properties. The method in which the elastic nature is retained is by fixing the fleecy padding layer to the elastic fabric whilst the latter is under tension, in its stretched form. Without this pre-stretch stage the padding fabric would tend to lock up the elasticity.

In the making of a nonwoven material layer, such as a padding layer of the invention, by needle punching, a batt of fibres is needled. This batt or mass of fibres can be formed by a variety of methods but typically a card / crossfolder route is by far the most common. A batt produced by this method would comprise several layers of a carded web, the number of which would be chosen in order to give the final area weight after needling. The action of needlepunching mechanically entangles the fibres by transferring a portion of fibre length which have been caught with a barb of a needle as it passes through successive web layers on its down stroke. This process happens the length and width of the needle board which usually contain thousands of needles-multipied by the number of downstrokes per minute. The resultant tufts of fibres created on the reverse side are often called needle plugs. In the making of a two layer bandage the conventional approach would be to needle the layers of the batt to the elastic layer in one step. However, surprisingly it was found that when a batt was needled directly to the elastic fabric the fibre transferred was too great when the desired gauge was attained. Consequently it was necessary to pre-needle the padding layer material prior to needling the padding layer to the elastic layer. It is believed that when the padding layer has been pre-needled the majority of fibres are already in some way mechanically entangled preventing excessively thick plugs of fibre being sent through the fabric.

According to the first aspect of the invention there is provided a method of making a two layer bandage according to claim 1

According to a further aspect of the invention the fixing of the filaments in the nonwoven layer is solely via needling and the fixing of the nonwoven layer to the elastic layer is solely by needling.

According to a further aspect of the invention the fixing of the filaments in the nonwoven layer and the fixing of the nonwoven layer to the elastic layer is not via heat-induced fusion of filaments.

According to a further aspect of the invention the fixing of the nonwoven material to the elastic layer does not comprise needling at a plurality of locations spaced a distance from one another.

Examples of needling at a plurality of locations spaced a distance from one another can be found in US 4,446,189 wherein needling is disclosed in a series of lines, a series of zig-zag lines, a series of wavy lines and a number of groups of spots wherein each set of spots is separated from adjacent sets of spots.

For the avoidance of doubt it should be understood in the needling of bandages of the invention care is taken to avoid any transverse line effect, for example by ensuring the advance per cycle is different from the needle spacing. However, since the needling entails reciprocating up and down at a fixed speed in conjunction with a fixed through-put speed it is unavoidable that some repetitive needle points are created. Therefore, the wording 'does not comprise needling at a plurality of locations' means that needling does not form specific areas of needling with significant spaces between these specific areas of needling, such significant spacing for example as shown in US 4,446,189. In addition, the wording 'continuous needling across the bandage' does not exclude a transverse line effect with very small gaps between the lines which may or may not be visible to the naked eye, but does exclude significant gaps between such lines.

According to a further aspect of the invention the fixing of the nonwoven material to the elastic layer comprises a continuous needling across the bandage, for example using a needling density as defined below..

According to a further aspect of the invention there is provided a method of the invention which further comprises releasing the tension on the elastic material and allowing the material to return to, or close to, its unelongated configuration,

It should be noted that in methods of the invention the bandage is needled from the nonwoven side with the plugs projecting from the elastic side.

Suitably said two layer bandage comprises an inner nonwoven padding layer and an outer elastic layer.

The term 'nonwoven' as used herein refers to a fabric-like material made from long fibres, bonded together by chemical, mechanical, heat or solvent treatment, for example by needling. In general fibrous material has been bonded without the need to be converted to a yarn (be it a staple yarn or filament) first, as is the case with traditional textile manufacture such as weaving and knitting Nonwoven materials may be formed by a variety of processes such as melt blowing, spun bonding processes, carding, needle punched web making processes, air laid web making processed, wet laid web making processes film aperture processes, staple fibre carding processes, hydro-entanglement etc.

Nonwoven materials may be formed from a variety of fibres from regular type fibres like viscose, polyester, acrylic and cotton fibres to the high performance type like Kevlar™, Nomex™, polybenzimidazole (PBI) or the exotic Alginate and Chitosan. In a further embodiment the nonwoven materials may be formed from a variety of fibres selected from viscose, polyester, acrylic, polypropylene and cotton fibres.

Nonwoven materials can be provided in different weights ranging from about 5 about 1500 g/m2 (gsm). Generally 5-30 gsm grades would be flat and used as coverstock applications, 30-60 gsm could be flat or lofty and could be used in a myriad of industries and applications. 60-1500 gsm could be needle-punched products again for a multitude of applications. Generally nonwoven materials used in the present invention are in the range about 60 to about 100 gsm, for example about 70 to about 90 gsm, for example about 75 to about 85 gsm, such as about 80 gsm.

Non woven materials when fixed by needling can be fixed with different needling densities. For example from lightly tacked orthopaedic padding grades as low as 15 needles/cm2 - to filter bag grades which may have needles densities of several hundred needles/cm2. For purposes of this invention, for preneedling the padding layer, a needle density not greater than about 50 needles/cm2 is preferred, for example about 10 to about 50/cm2, more preferably around about 25 needles/cm2, for example about 15 to about 35 needles/cm2, alternatively about 20 to about 30 needles/cm2, alternatively about 25 to about 30 needles/cm2, alternatively about 25 to about 27 needles/cm2., alternatively about 26 to about 27 needles/cm2, such as about 26.5 needles/cm2. For needling the elastic layer to the padding layer a needle density in the range of about 40 to about 80 needles/cm2 would be preferred. For example about 50 to about 70 needles/cm2, alternatively about 55 to about 65 needles/cm2, alternatively about 60 to about 70 needles/cm2, such as about 66.9 needles/cm2.

Nonwoven materials when fixed by needling can be facilitated by a number of different designs of barbed needles and penetration depths. Suitable needles are any needles which can facilitate the mechanical entanglement of a fibrous batt to form a nonwoven. Examples of suitable needles include 15x18x38 RB (Groz-Beckert AG, Albstadt, Germany).

Various gauge needles can be used for orthopaedic grades of nonwoven material the gauges varying from coarse 32 gauge to fine gauges for example 42 gauge. In the present invention gauges of 38 or less are preferred, for example a gauge in the range 32 to 38, such as 33 to 37, 34 to 36 or 36 to 38. Gauges of 38 or less gave plugs which were more aesthetically pleasing and more comfortable against the skin. Suitable penetration depths for needling the nonwoven layer are in the range 20-22mm, for example, 20 mm. In a further embodiment the suitable penetration depths are in the range about 20 to about 25mm.

Needles suitable for the needling of the nonwoven material would also be suitable for the needling of the padding layer to the elastic layer, although different gauge needles can be used, for example coarser needles, for example in the range 42-30, such as 32-42, 34 to 42, 36 to 42, 36 to 40 or 38 to 40 gauge.

The term 'elastic layer' as used herein refers to any material which has a stretch capability in it's longitudinal direction. For example a stretch capability between 5% and 200% wherein a 100% stretch relates to a doubling in length of the material such as between 100 and 160% and between 120% and 140%.. Examples of suitable elastic layers include a fabric comprising mainly cotton and a smaller part of Polyurethane-yarns to reach an elasticity between 100% and 160%, for example between 120% and 140%.

In the method of the invention the elastic layer is presented (or "fed") under tension to the preneedled nonwoven layer and maintained at that tension until needled. That way when the tension is released, the stretched fabric recovers to its starting length. If the fabric was not pre-stretched prior to combining with the pre-needled nonwoven layer the needling would tend to lock-up and hinder the stretching required in use. The degree of stretch necessary prior to needling is related to the force required to extend the bandage and this can vary for the stretch bandage construction- it is this force which ultimately relates to the compression exerted.

During the designing of the method of the invention it was found that at some penetrations, although the bandage, was functional the plugs were unsightly and the bandage felt rough. The conventional approach to this problem would be to decrease the size of the plugs so they barely protruded the reverse layer. However, when this point was reached there was insufficient entanglement such that the fleecy fabric could be separated from the stretchy fabric too easily. Contrary to expectation increasing the number of plugs by increasing the needle density failed to counter the problem. Surprisingly, it was found that by substantially increasing the size of the plugs actually solved the problem. The use of relatively high penetration depths resulted in protruding fibre plugs which were sufficiently long enough to bend over rather than act in a stiffer bristle form.

The efficacy of fixing of the padding layer to the elastic layer is dependent on both the needle penetration depth and the needling density. Within certain limits a lower needle penetration depth can be compensated for by a higher needling density.

The penetration depth of the needles for the needling of the padding layer to the elastic layer is the needle depth which prevents delamination, i.e. separation of the padding and elastic layers, when combined with the needling density. Penetration depths are partly dependent on the specific needle used, for example for a 15x18x38 RB needle (Groz-Beckert AG, Albstadt, Germany) was in the range from 15 - 25 mm, for example 20 - 23mm, such as 21.5 mm - resulting in a pile on the bandage comprising plugs between 2 to 20 mm in lenght, for example 2 to 15 mm in length. In one embodiment the bandage comprises plugs of about 2 to about 5 mm.

It should be noted that design of the needle will impact the required penetration depth. For example, needles with regularly shaped barbs tend to require deeper penetration depths than needles with high density barbs. In general needles with regular barb spacing give a more pleasing visual effect than needles with higher density barbs.

The needling density for the fixing of the padding layer to the elastic layer is any needling density which prevents delamination, i.e. separation of the padding and elastic layers, when combined with the penetration depth. Suitable needling densities are between about 40 to about 80 needles per cm².

In addition to fixing the padding layer to the elastic layer using needling, optionally an adhesive layer can also be used. Adhesives suitable for fixing bandage materials together are well known in the art. Examples include: pressure sensitive adhesives, hot melt adhesives (including scattercoat powders, web adhesives , dot coatings, melt spray adhesives etc), emulsion adhesives, solution adhesives and solvent adhesives.

In order to practice methods of the invention a conventional needling machine was modified. Said modified machine forms a further aspect of the invention.

There is provided an apparatus for manufacturing a product, which product comprises a nonwoven material, such as a padding bandage grade non-woven material, attached to an elastic material and wherein the apparatus comprises means adapted to place the elastic material under tension and to attach said nonwoven material to said elastic material whilst the elastic material is under tension.

Suitably, the product comprises a bandage.

The apparatus may be adapted to apply tension to said elastic material as the nonwoven material is attached thereto. Suitably, the apparatus is adapted to apply tension to said elastic material before said nonwoven material is attached thereto.

Suitably, the apparatus is adapted to attach said nonwoven material to said elastic material whilst the elastic material is held under tension.

Suitably, the apparatus is adapted to attach said nonwoven material to said elastic material whilst the nonwoven material is held under substantially no tension.

Suitably, the apparatus is adapted to place the elastic material under tension such that it is in an elongated configuration when the padding material is attached thereto.

Suitably, the apparatus is adapted to draw a web of elastic material from a supply means. Suitably, the supply means comprises a roll of elastic material. Suitably, the supply means comprises a let-off stand/roll.

The apparatus may be adapted to tension the web of elastic material at a point downstream of the supply means. The apparatus may be adapted to tension the web as it is drawn from the supply means. Suitably, the web is drawn from the supply means substantially without being tensioned and is tensioned at a downstream point.

Suitably, the apparatus is adapted to apply tension to a web of elastic material to stretch the web. Suitably, the apparatus is adapted to stretch the web substantially in its direction of travel. Suitably, the apparatus is adapted to apply tension to a web of elastic material to elongate the web.

Suitably, the apparatus is adapted to stretch the elastic material such that the material is stretched by at least 50%, for example by 100% or more when the nonwoven material is attached thereto. Suitably, the % stretch is measured as a % of the unstretched length of the elastic material.

Suitably, the apparatus is adapted to lay nonwoven material onto a web of elastic material.

Suitably, the apparatus is adapted to hold the web of elastic material under tension at a stage at which the nonwoven material is laid onto the web of elastic material.

Suitably, the apparatus is adapted to hold a web of elastic material under tension at a stage at which it is attached to the nonwoven material.

The apparatus may be adapted to attach fibres to an elastic material such that a nonwoven material is formed as it is attached to the elastic material. The apparatus may be adapted to attach a batt of fibres to the elastic material. Suitably, the apparatus is adapted to attach a batt of fibres which has been needled to the elastic material. Suitably, the apparatus is adapted to attach an already formed nonwoven material to the elastic material.

The apparatus may be adapted to needle fibres to an elastic material such that a nonwoven material is formed as it is attached to the elastic material. Suitably, the apparatus is adapted to needle an already formed nonwoven material to the elastic material.

Suitably, the apparatus is adapted to attach a pre-needled nonwoven material to the elastic material. Suitably, the apparatus is adapted to needle fibres to form a nonwoven material upstream of the stage at which the nonwoven material is attached to the elastic material. Suitably, the apparatus is adapted to needle a nonwoven material at stage upstream of the stage at which the nonwoven material is attached to the elastic material such that a pre-needled nonwoven material is attached to the elastic material. Suitably, the pre-needled nonwoven material is needled to the elastic material such that the nonwoven material is needled twice by the apparatus.

The apparatus may be adapted to create a web of nonwoven material at a point upstream of the stage at which it is attached to the elastic material. The apparatus may be adapted to needle fibres to create a web of nonwoven material at a point upstream of the stage at which it is attached to the batt. Alternatively, the apparatus may be adapted to needle fibres to create a web of nonwoven material and the nonwoven material produced may then be passed through the apparatus again to attache it to the elastic material.

The apparatus may comprise a first needling station for forming a web of nonwoven material and a second needling station downstream of the first needling station for needling the web of nonwoven material to the web of elastic material whilst the web of elastic material is under tension. Alternatively, the apparatus may comprise a single needling station which may be used for forming a web of nonwoven material and through which said nonwoven material may be passed following formation for needling the web of nonwoven material to the web of elastic material whilst the web of elastic material is under tension.

Suitably, the or each needle station comprises a needleloom.

Suitably, the apparatus is adapted to lay a web of nonwoven material onto a web of elastic material.

Suitably, the apparatus is adapted to hold the web of elastic material under tension at a stage at which the web of nonwoven material is laid onto the web of elastic material.

Suitably, the apparatus is adapted to hold a web of elastic material under tension at a stage at which it is attached to the web of nonwoven material.

Suitably, the apparatus is adapted to attach the elastic material to the nonwoven material by needling. Suitably, the apparatus comprises a needling means. Suitably, the apparatus is adapted to hold the elastic material under tension as the nonwoven material is needled thereto.

Suitably, the apparatus is adapted to relax the tension applied to the web of elastic material once it has been attached to the nonwoven material.

Suitably, the apparatus is adapted to produce a fabric in a continuous process.

Suitably, the apparatus comprises tensioning means for placing the web of elastic material under tension.

The apparatus is suitably adapted to convey the web of elastic material through the apparatus such that it is placed under tension during part of its conveyance through the apparatus and is under tension when the non woven material is attached thereto.

Suitably, the apparatus comprises means upstream and downstream of the attachment station for gripping the web of elastic material such that the web of elastic material extending therebetween is held in tension.

The apparatus may be adapted to tension the web of elastic material by having a differential between input and output speeds of the web of elastic material.

Suitably, the input and output speeds of the apparatus are tailored to give the desired percentage stretch to the elastic material.

Suitably, the apparatus comprises first feeding means for feeding and/or drawing a web of elastic material from a supply means. Suitably, the apparatus comprises second feeding means for feeding a web of elastic material to and/or through an attachment station at which nonwoven material is attached thereto. Suitably, the second feeding means is arranged to feed the web at a faster rate than the first feeding means such that the section of web between the first and second feeding means is placed under tension and stretched.

Suitably, the apparatus comprises a powered nip. The apparatus may comprise a mangle as the first feeding means. Suitably, the apparatus comprises driven rollers for drawing a web of elastic material from a supply means, suitably from a let-off stand. Suitably, the apparatus comprises two driven rollers biased towards one another. Suitably, the apparatus comprises two driven rollers pushed towards one another by pneumatic cylinders. The driven rollers may have rubber surfaces for contacting the material.

Suitably, the apparatus comprises an output drive. Suitably, the apparatus comprises an output drive downstream of the attachment station. Suitably, the apparatus comprises a needleloom having an output drive. Suitably, the output drive comprises output rollers.

Suitably, the apparatus comprises a powered nip having driven rollers which are adapted to prevent slippage of the elastic material and which thus stop an output drive of the needleloom pulling the material through the rollers at the output speed. The material may thus be held under tension between the loom output rollers and the powered nip rollers.

Alternatively, the apparatus may use other means to place the elastic material under tension. The apparatus may for example comprise a brake, such a torque controlled brake, for example a magnetic particle brake. on the let-off roller, a load controlled clutch let-off or a speed differential system. The torque force may be determined by the load elongation characteristics.

A braking system may make use of a brake which allows tension to be generated between the attachment station and the let-off roller.

A load controlled clutch may be in the form of a magnetic clutch which can be preset to a certain load force. Said load force may be predetermined by the load elongation characteristics of the elastic fabric to give the desired amount of stretch.

Suitably, the apparatus comprises a conveyor to support the web of elastic material and the web of nonwoven material as they are fed to the attachment station.

The apparatus may be adapted to form a batt from staple fibres. The apparatus may comprise a carding and cross-folder means for forming a batt from staple fibres. Suitably the apparatus may comprise an airlaid batt of staple fibre. Suitably, the apparatus is adapted to needle a batt to form a nonwoven material. Suitably, the apparatus is adapted to feed the needled batt to an attachment station

Suitably, the apparatus comprises a doffer. Suitably, the apparatus comprises a cross folder.

The apparatus may be adapted to lay a web down multiple times on a slow moving lattice conveyor. Multiple layers of the batt may be attached to a single layer of elastic material at the attachment station. Suitably, multiple layers of the batt are needled to one another to form a batt which is then attached to a single layer of elastic material at the attachment station.

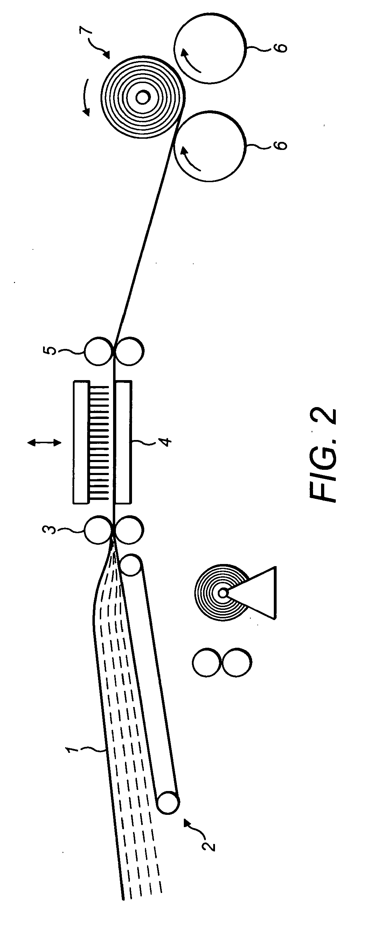

As illustrated in Figure 2 an apparatus for manufacturing the bandage fabric comprises a let off roller 8 carrying a supply of elastic material. The apparatus conveys a web of elastic material 100 from the let off roller 8 to a needling station (attachment station) 101 which comprises a needle loom. Also conveyed to the needling station 101 is a batt 102. The apparatus is adapted to needle the nonwoven material to the elastic material at the needling station 101 whilst the web of elastic material 100 is under tension. The bandage formed at the needling station 101 is then conveyed to the output drive rollers 103 of the apparatus. Between the let off roller 8 and the output drive 103 is a variable speed mangle with pneumatic nip 9. The mangle 9 is downstream of the needling station 101 and comprises a powered mangle which is configured to draw the web of elastic material from the let off roller 8 at a rate which is slower than the rate at which the output drive 103 feeds the web of elastic material to the needling station 101. The speed differential thus causes the web of elastic material between the mangle 9 and output drive 103 to be placed under tension and so stretched. The stretched web of elastic material 100 is fed over a conveyor 104 which supports the web of elastic material and the batt of nonwoven material which is laid onto it. Alternatively, the web of elastic material is fed directly to the input rollers The web of elastic material is held in an elongated configuration when the batt of nonwoven material is laid onto it and also when the web of nonwoven material is attached to it. When the fabric passes the output drive 103 the tension is largely released with some delay in regaining its elasticity and the web of elastic material returns towards its original unelongated configuration. The density of the nonwoven material per unit length of the bandage after the output drive 103 is thus greater than was the density of the nonwoven material per unit length of the web of elastic material when they were attached at the needling station 101.

Two layer bandages according to claim 6 represent a further aspect of the invention. According to a further aspect there is provided a two layer bandage comprising:
(i) a padding layer comprising a nonwoven material; and
(ii) an elastic layer,
characterized in that the elastic layer has a pile comprising plugs up to about 20mm in length.

According to a further aspect of the invention there is provided a two layer bandage comprising:
(i) a padding layer comprising a nonwoven material; and
(ii) an elastic layer,
characterized in that the elastic layer has a pile comprising plugs up to about 3 mm from the outer surface of the elastic layer and/or about 5-8 mm from the outer surface of the nonwoven layer.

According to a further aspect of the invention there is provided a two layer bandage as defined above further comprising one or more of the following:
(a) the fixing of the filaments in the nonwoven layer is solely via needling and the fixing of the nonwoven layer to the elastic layer is solely by needling;
(b) the fixing of the filaments in the nonwoven layer and the fixing of the nonwoven layer to the elastic layer is not via heat-induced fusion of filaments;
(c) the fixing of the nonwoven material to the elastic layer does not comprise needling at a plurality or locations spaced a distance from one another;
(d) the fixing of the nonwoven material to the elastic layer comprises a continuous needling across the bandage, for example using a needling density as defined herein.

According to a further aspect there is provided a two layer bandage comprising:
(i) a padding layer comprising a nonwoven material; and
(ii) an elastic layer,
comprising one or more of the following
(a) the fixing of the filaments in the nonwoven layer is solely via needling and the fixing of the nonwoven layer to the elastic layer is solely by needling;
(b) the fixing of the filaments in the nonwoven layer and the fixing of the nonwoven layer to the elastic layer is not via heat-induced fusion of filaments;
(c) the fixing of the nonwoven material to the elastic layer does not comprise needling at a plurality or locations spaced a distance from one another;
(d) the fixing of the nonwoven material to the elastic layer comprises a continuous needling across the bandage, for example using a needling density as defined herein.

Suitably said two layer bandage comprises an inner nonwoven padding layer and an outer elastic layer.

In one embodiment the nonwoven material has an intrinsic needling density of about 25 to about 28 needles/cm2, such as about 26 to about 27 needles/cm2, for example about 26.5 needles/cm2.

In one embodiment the padding layer and the elastic layer have a needling density of about 60 to about 70 needles/cm2, such as about 66.9 needles/cm2.

In a further embodiment of the invention there is provided a two layer bandage as defined above with a pile comprising plugs between about 2 to about 15 mm in length.

In a further embodiment of the invention there is provided a two layer bandage as defined above with a pile comprising plugs up to about 15 mm in length. In a further embodiment there is provided a two layer bandage as defined above with a pile comprising plugs up to about 6 mm in length from the outer surface of the nonwoven layer.

In a further embodiment of the invention there is provided a two layer bandage as defined above with a pile comprising plugs up to about 20mm in length. In a further embodiment of the invention there is provided a two layer bandage as defined above with a pile comprising plugs up to about 8mm from the outer surface of the nonwoven layer.

The term 'about' in this application relates to ±2mm, ±1mm or ±0.5mm when used in relation to distances measured in millimeters. The term 'about' when used with respect to other parameter, such as needling density, means ±10% of the value or ±5% of the value or ±2% of the value or ±1 % of the value..

Bandages of the invention are suitable for a number of applications, particularly for use as orthapaedic bandages.

The invention also provides as a further aspect of the invention the use of bandages of the invention in therapy. Thus, as a fourth aspect of the invention there is provided the use of a bandage of the invention in therapy, for example compression therapy.

Compression therapy is known for use in the treatment of oedema and other venous and lymphatic disorders, for example, of the lower limbs. An area of particular use is in the management and/or treatment of chronic wounds, such as venous leg ulcers.

According to a further aspect of the invention there is provided a bandage of the invention for the management and/or treatment of chronic wounds.

According to a further aspect of the invention there is provided the use of a bandage of the invention in the manufacture of a medicament for the management and/or treatment of chronic wounds.

According to a further aspect of the invention there is provided a method for the management/or and treatment of chronic wounds using a bandage of the invention.

The invention further comprises a method of forming a compression binding *in-situ* wherein a bandage of the invention is wrapped around a patient's body, such as a leg with varicose ulcers and maintained in position in compression therein. The compression may be in the range 20-60mm Hg in the supine position of the leg after wrapping, specially between 40-60 mmHg at an ABPI (Ankle Brachial Pressure Index) > 0.8.

The application will now be exemplified by the following non-limiting example in which:
- Figure 1: shows a schematic view of the machinery used to preneedle the padding layer.
- Figure 2: shows a schematic view of the machinery used to needle the preneedled padding layer to the elastic layer.

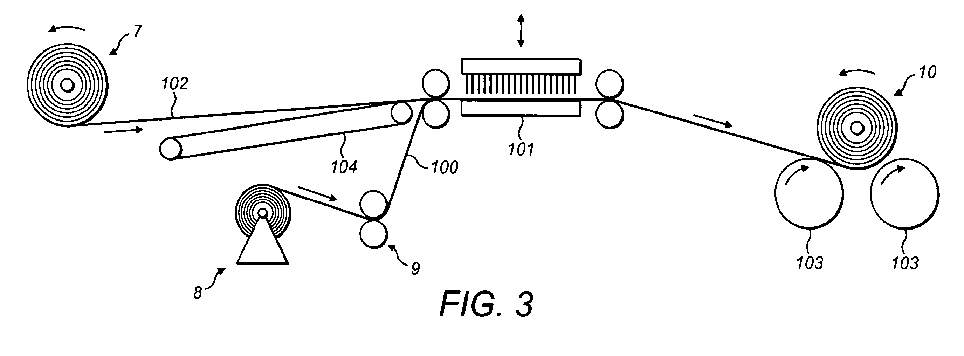

- Figure 3: shows a variation on the machinery shown in Figure 2 wherein the let off unit comprising the stand (8) was switched around such that the fabric passed directly to the needleloom input rollers.

In these diagrams the following integers are labeled:
(1) fibrous batt comprising the multiple layers of the carded viscose web;
(2) needleloom input lattice / conveyor;
(3) needleloom input rollers;
(4) needleloom, comprising reciprocating needleboard above a perforated bedplate;
(5) needleloom output rollers;
(6) surface driven wind- up rollers;
(7) roll of pre-needled batt;
(8) let - off stand;
(9) variable speed mangle with pneumatic nip;
(10) dual layer bandage of the invention.

### Example 1

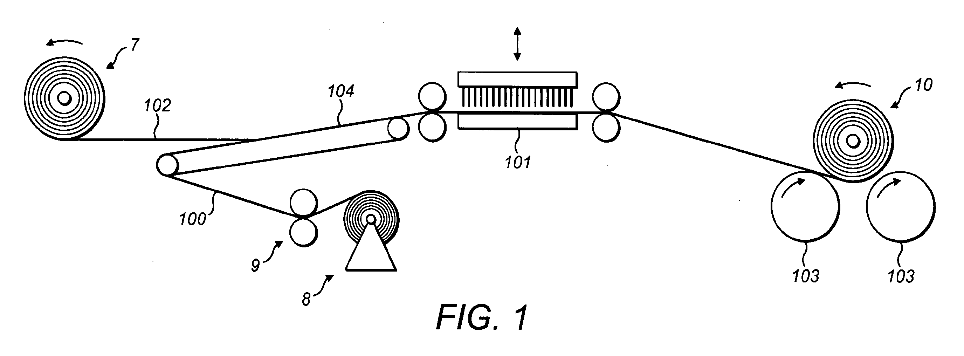

Using a conventional hopper fed card cross-folding line, an 80 gsm cross folded batt of 100 % 3.1 decitex 40 mm viscose (Lenzing Fibers Grimsby Ltd, Grimsby, UK) was produced at 200 cm width. This was then needled using a Dilo 0-11/40 needleloom (1971 model) (Oskar Dilo Maschinenfabrik KG, Eberbach, Germany. The needle density used was 26.54 needles/cm² using a loom speed of 490 RPM with a throughput speed of 7.2 m/ min. The needles used were Groz Beckert 3 inch long needles: 15 x18 x38 RB (Groz-Beckert AG, Albstadt, Germany). The penetration used was 20 mm.(see Figure 1)

200 m rolls of this pre-needled batt were produced.

With the crossfolder stopped, these rolls were then let-off back through the needlelloom where they were needled to the pre stretched elastic bandage fabric. The throughput speed was this time dropped to 3.4 m/min to give a higher needle density. The needle density used was 66.8 needles /cm² using a loom speed of 583 RPM. The same Groz beckert needles were used as the pre- needled roll stage but the penetration used was 21.5 mm.(see Figure 2)

The elastic fabric comprised a woven fabric 115 gsm in weight, with a fibre composition of 93 % cotton, 5 % polyamide and 2 % polyurethane, with an the elasticity of 150 % +/- 20 %.

The elastic fabric was placed on a let off stand under the input lattice of the needleloom as is common for scrims. Unlike a scrim this elastic fabric was then powered from the let - off stand using a variable speed powered mangle or nip roller arrangement. This equipment was specially designed and built for this process and allowed the let-off speed to be controlled relative to the input speed of the needleloom (see Figure 2). In this case, the let- off speed was set to half the input speed thus giving 100 % stretch. This pre-stretch allowed the needled composite not to be hindered from stretching by the fibre needled into it.

The degree of stretch could be checked on the input lattice by measuring between set marks printed on the edge of the elastic fabric.

Once needled the "composite" material was wound up on two surface driven rollers. The speed of these rollers were the same as the input section which did prevent the fabric restoring its former state until subsequently run-off.

## Claims

1. A method of making a two layer bandage comprising a nonwoven padding layer and an elastic layer,
said method comprising
(i) providing a pre-needled nonwoven padding layer formed from fibres and an elastic layer, said elastic layer under tension and said nonwoven padding layer not under tension, and
(iii) fixing the nonwoven layer to the elastic layer by needling.

2. A method according to Claim 1 wherein the fixing of the nonwoven material to the elastic layer comprises a continuous needling across the bandage.

3. A method according to claims 1 or 2 wherein the fixing of the fibres in the nonwoven layer is solely via needling and the fixing of the nonwoven layer to the elastic layer is solely by needling.

4. A method according to any one of the preceding claims wherein the elastic layer is tensioned with a stretch between about 50 to about 120%.

5. A method according to any one of the preceding claims wherein the needle penetration depth for fixing the nonwoven material to the elastic layer is about 20 to about 23mm.

6. A two layer elastic bandage comprising:
a padding layer comprising a nonwoven material formed from fibres and an elastic layer,
**characterized in that** the fixing of the fibers in the nonwoven padding layer is solely by needling and the fixing of the nonwoven layer to the elastic layer is solely by needling and wherein the bandage is obtained by the method according to any one of claims 3 to 5.

7. A two layer elastic bandage according to Claim 6 wherein the bandage comprises a pile with plugs up to about 8 mm in length measured from the outer surface of the nonwoven layer.

8. A two layer elastic bandage according to Claims 6 or 7 wherein the bandage comprises a pile with plugs up to about 3 mm in length measured from the outer surface of the elastic layer.

9. A two layer elastic bandage according to any one of Claims 6 to 8 wherein the needling density for the fixing of the padding layer to the elastic layer is between about 40 to about 80 needles per cm².

10. A two layer elastic bandage according to any one of Claims 6 to 9 wherein preneedling of the nonwoven padding layer has a needling density of about 15 to about 35 needles/cm².

11. A method according to any one of Claims 1 to 5 or a bandage according to any one of Claims 6 to 10 wherein the padding layer comprises viscose, polyester, acrylic, polypropylene and/or cotton fibres.

12. A method according to any one of Claims 1 to 5 or a bandage according to any one of Claims 6 to 10 wherein the padding layer has a weight between about 60 to about 100 gsm.

13. A method according to any one of Claims 1 to 5, or a bandage according to any one of Claims 6 to 10 wherein the padding layer has a preneedling density of about 10 to about 50 needles/cm².

14. A method according to any one of Claims 1 to 5 or a bandage according to any one of Claims 6 to 10 wherein the padding layer is fixed to the elastic layer with a needling density of about 50 to about 70 needles/cm².

15. A bandage according to any one of Claims 6 to 10 for use in compression therapy.

## Patentansprüche

1. Verfahren zur Herstellung einer zweilagigen Bandage, umfassend eine Vliesstoff-Wattierungsschicht und eine elastische Schicht,
welches Verfahren enthält:
(i) Vorsehen einer aus Fasern gebildeten vorvernadelten Vliesstoff-Wattierungsschicht und einer elastischen Schicht, wobei die elastische Schicht unter Spannung ist und die Vliesstoff-Wattierungsschicht nicht unter Spannung ist, und
(iii) Befestigen der Vliesstoffschicht an der elastischen Schicht durch Vernadelung.

2. Verfahren nach Anspruch 1, wobei das Befestigen des Vliesstoffmaterials an der elastischen Schicht eine kontinuierliche Vernadelung über die Bandage umfasst.

3. Verfahren nach den Ansprüchen 1 oder 2, wobei das Befestigen der Fasern in der Vliesstoffschicht alleine durch Vernadelung erfolgt und das Befestigen der Vliesstoffschicht an der elastischen Schicht alleine durch Vernadelung erfolgt.

4. Verfahren nach einem der vorhergehenden Ansprüche, wobei die elastische Schicht mit einer Dehnung zwischen etwa 50 bis etwa 120% gespannt wird.

5. Verfahren nach einem der vorhergehenden Ansprüche, wobei die Nadeldurchdringungstiefe zur Befestigung des Vliesstoffmaterials an der elastischen Schicht etwa 20 bis etwa 23 mm beträgt.

6. Zweilagige elastische Bandage, umfassend:
eine Wattierungsschicht, die ein aus Fasern gebildetes Vliesstoffmaterial enthält, und eine elastische Schicht,
**dadurch gekennzeichnet, dass** die Befestigung der Fasern in der Vliesstoff-Wattierungsschicht alleine durch Vernadelung erfolgt und die Befestigung der Vliesstoffschicht an der elastischen Schicht alleine durch Vernadelung erfolgt, und wobei die Bandage durch das Verfahren nach einem der Ansprüche 3 bis 5 erhalten wird.

7. Zweilagige elastische Bandage nach Anspruch 6, wobei die Bandage einen Flor mit Zapfen mit einer Länge bis zu etwa 8 mm, gemessen von der äußeren Oberfläche der Vliesstoffschicht, aufweist.

8. Zweilagige elastische Bandage nach Anspruch 6 oder 7, wobei die Bandage einen Flor mit Zapfen mit einer Länge bis zu etwa 3 mm, gemessen von der äußeren Oberfläche der elastischen Schicht, aufweist.

9. Zweilagige elastische Bandage nach einem der Ansprüche 6 bis 8, wobei die Vernadelungsdichte zur Befestigung der Wattierungsschicht an der elastischen Schicht zwischen etwa 40 bis etwa 80 Nadeln pro cm² beträgt.

10. Zweilagige elastische Bandage nach einem der Ansprüche 6 bis 9, wobei die Vorvernadelung der Vliesstoff-Wattierungsschicht eine Vernadelungsdichte von etwa 15 bis etwa 35 Nadeln/cm² beträgt.

11. Verfahren nach einem der Ansprüche 1 bis 5 oder Bandage nach einem der Ansprüche 6 bis 10, wobei die Wattierungsschicht Viskose-, Polyester-, Acryl-, Polypropylen- und/oder Baumwollfasern enthält.

12. Verfahren nach einem der Ansprüche 1 bis 5 oder Bandage nach einem der Ansprüche 6 bis 10, wobei die Wattierungsschicht ein Gewicht zwischen etwa 60 bis etwa 100 g/m² hat.

13. Verfahren nach einem der Ansprüche 1 bis 5 oder Bandage nach einem der Ansprüche 6 bis 10, wobei die Wattierungsschicht eine Vorvernadelungsdichte von etwa 10 bis etwa 50 Nadeln/cm² hat.

14. Verfahren nach einem der Ansprüche 1 bis 5 oder Bandage nach einem der Ansprüche 6 bis 10, wobei die Wattierungsschicht an der elastischen Schicht mit einer Vernadelungdichte von etwa 50 bis etwa 70 Nadeln/cm² befestigt wird.

15. Bandage nach einem der Ansprüche 6 bis 10 zur Verwendung in der Kompressionstherapie.

## Revendications

1. Procédé de fabrication d'un bandage à deux couches comprenant une couche de rembourrage non tissée et une couche élastique,
ledit procédé comprenant:
(i) la fourniture d'une couche de rembourrage non tissée pré-aiguilletée formée à partir de fibres et d'une couche élastique, ladite couche élastique étant sous tension et ladite couche de rembourrage non tissée n'étant pas sous tension, et
(iii) la fixation de la couche non tissée sur la couche élastique par aiguilletage.

2. Procédé selon la revendication 1, dans lequel la fixation du matériau non tissé sur la couche élastique comprend un aiguilletage continu sur l'ensemble du bandage.

3. Procédé selon les revendications 1 ou 2, dans lequel la fixation des fibres dans la couche non tissée se fait uniquement par l'intermédiaire d'un aiguilletage et la fixation de la couche non tissée sur la couche élastique se fait uniquement par un aiguilletage.

4. Procédé selon l'une quelconque des revendications précédentes, dans lequel la couche élastique est tendue avec un étirement compris entre environ 50 et environ 120 %.

5. Procédé selon l'une quelconque des revendications précédentes, dans lequel la profondeur de pénétration de l'aiguille pour fixer le matériau non tissé sur la couche élastique est comprise entre environ 20 et environ 23 mm.

6. Bandage élastique à deux couches comprenant:
une couche de rembourrage comprenant un matériau non tissé formé à partir de fibres et d'une couche élastique,
**caractérisé en ce que** la fixation des fibres dans la couche de rembourrage non tissée se fait uniquement par un aiguilletage et la fixation de la couche non tissée sur la couche élastique se fait uniquement par un aiguilletage et dans lequel le bandage est obtenu grâce au procédé selon l'une quelconque des revendications 3 à 5.

7. Bandage élastique à deux couches selon la revendication 6, dans lequel le bandage comprend un tissu à boucles avec des touffes pouvant atteindre jusqu'à environ 8 mm de longueur, mesurées à partir de la surface externe de la couche non tissée.

8. Bandage élastique à deux couches selon les revendications 6 ou 7, dans lequel le bandage comprend un tissu à boucles avec des touffes pouvant atteindre jusqu'à environ 3 mm de longueur, mesurées à partir de la surface externe de la couche élastique.

9. Bandage élastique à deux couches selon l'une quelconque des revendications 6 à 8, dans lequel la densité d'aiguilletage pour la fixation de la couche de rembourrage sur la couche élastique est comprise entre environ 40 et environ 80 aiguilles par cm².

10. Bandage élastique à deux couches selon l'une quelconque des revendications 6 à 9, dans lequel le pré-aiguilletage de la couche de rembourrage non tissée a une densité d'aiguilletage comprise entre environ 15 et environ 35 aiguilles/cm².

11. Procédé selon l'une quelconque des revendications 1 à 5 ou bandage selon l'une quelconque des revendications 6 à 10, dans lequel la couche de rembourrage comprend des fibres de viscose, de polyester, d'acrylique, de polypropylène et/ou de coton.

12. Procédé selon l'une quelconque des revendications 1 à 5 ou bandage selon l'une quelconque des revendications 6 à 10, dans lequel la couche de rembourrage a un poids compris entre environ 60 et environ 100 g/m².

13. Procédé selon l'une quelconque des revendications 1 à 5 ou bandage selon l'une quelconque des revendications 6 à 10, dans lequel la couche de rembourrage a une densité de pré-aiguilletage d'environ 10 à environ 50 aiguilles/cm².

14. Procédé selon l'une quelconque des revendications 1 à 5 ou bandage selon l'une quelconque des revendications 6 à 10, dans lequel la couche de rembourrage est fixée à la couche élastique avec une densité d'aiguilletage d'environ 50 à environ 70 aiguilles/cm².

15. Bandage selon l'une quelconque des revendications 6 à 10 destiné à être utilisé en thérapie par compression.
